(19) Europäisches Patentamt
European Patent Office
Office européen des brevets

(11) Veröffentlichungsnummer: **0 156 253**
**B1**

(12) # EUROPÄISCHE PATENTSCHRIFT

(45) Veröffentlichungstag der Patentschrift: **08.08.90**

(21) Anmeldenummer: **85102915.7**

(22) Anmeldetag: **14.03.85**

(51) Int. Cl.[5]: **B 01 J 31/40,** B 01 D 11/02, C 07 C 45/50, C 07 C 29/16, B 01 J 23/90

(54) **Verfahren zur Rückgewinnung von Rhodium aus Reaktionsprodukten der Oxosynthese.**

(30) Priorität: **26.03.84 DE 3411034**

(43) Veröffentlichungstag der Anmeldung:
**02.10.85 Patentblatt 85/40**

(45) Bekanntmachung des Hinweises auf die Patenterteilung:
**08.08.90 Patentblatt 90/32**

(84) Benannte Vertragsstaaten:
**AT DE FR GB IT NL SE**

(56) Entgegenhaltungen:
**DE-A-2 627 354**
**DE-A-3 135 127**
**US-A-4 292 196**

(73) Patentinhaber: **HOECHST AKTIENGESELLSCHAFT**
**Postfach 80 03 20**
**D-6230 Frankfurt am Main 80 (DE)**

(72) Erfinder: **Cornils, Boy, Dr. Dipl.-Chem.**
**Friedrich-Ebert-Strasse 45**
**D-4220 Dinslaken (DE)**
Erfinder: **Konkol, Werner, Dr. Dipl.-Chem.**
**Lützowstrasse 40a**
**D-4200 Oberhausen 11 (DE)**
Erfinder: **Bahrmann, Helmut, Dr. Dipl.-Chem.**
**Rohstrasse 48**
**D-4236 Hamminkeln-Brünen (DE)**
Erfinder: **Bach, Hanswilhelm, Dr. Dipl.-Chem.**
**Alleestrasse 56**
**D-4100 Duisburg 11 (DE)**
Erfinder: **Wiebus, Ernst**
**Ferdinandstrasse 77**
**D-4200 Oberhausen 11 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 156 253 B1

**Beschreibung**

Die vorliegende Erfindung betrifft ein verbessertes Verfahren zur Abtrennung und Rückgewinnung von Rhodium aus den Reaktionsgemischen der Oxosynthese.

Die Herstellung von Aldehyden und Alkoholen durch Anlagerung von Kohlenmonoxid und Wasserstoff an olefinische Doppelbindungen (Hydroformylierung) ist bekannt. Die Reaktion wird durch Metalle der 8. Nebengruppe der Periodensystems oder deren Verbindungen katalysiert, die unter den Reaktionsbedingungen Carbonyle oder Hydridocarbonyle bilden. Während früher Kobalt und Kobaltverbindungen als Katalysatoren ein gesetzt wurden, finden heute in zunehmendem Maße Rhodiumkatalysatoren Anwendung, obgleich Rhodium um ein Vielfaches teurer als Kobalt ist. Rhodium gelangt dabei allein oder in Kombination mit Komplexbildnern, z.B. organischen Phosphinen zum Einsatz. Während die Oxosynthese mit Rhodium als Katalysator Reaktionsdrucke von 250 bis 300 bar ($25 \cdot 10^3$ bis $30 \cdot 10^3$ kPa) erfordert, genügen bei Einsatz von Rhodium-Komplexverbindungen Drucke von 10 bis 50 bar ($1{,}0 \cdot 10^3$ bis $5{,}0 \cdot 10^3$ kPa).

Für Rhodium-Katalysatoren ergeben sich in vielen Fällen deutliche Vorteile. Sie besitzen höhere Aktivität und Selektivität und ermöglichen überdies einen komplikationsfreien Betrieb der Produktionsanlage, insbesondere hinsichtlich der Durchführung der Synthese und der Ausbringung der Produke aus dem Reaktor. Schließlich kann das klassische Oxoverfahren auf Basis von Kobaltkatalysatoren under Verwendung der vorhandenen Apparateteile in vielen Fällen mit nur geringen Investitionen auf Rhodiumkatalysatoren umgestellt werden.

Ein sehr bewährtes Verfahren zur Hydroformylierung von Olefinen ist in der DE—A2—26 27 354 beschrieben. Es beruht auf der Verwendung eines zweiphasigen Reaktionssystems, nämlich einer wäßrigen Phase, die den Katalysator enthält und einer organischen Phase, die im wesentlichen aus dem Einsatzolefin und dem Reaktionsprodukt gebildet wird. Gegenstand der DE—A—31 35 127 ist eine Variante des vorstehend beschriebenen Verfahrens, dessen Merkmal der Zusatz eines amphiphilen Reagenzes zum Reaktionssystem ist. Dadurch wird eine Herabsetzung der Reaktionszeit und eine Erhöhung des n/iso-Verhältnisses der Produktaldehyde erzeilt.

Est ist möglich auch mit Einsatz von Rhodium ohne Komplexbildner als Hydroformylierungskatalysator zu arbeiten. Allerdings bereitet dann die annähernd verlustfreie Abtrennung und Wiedergewinnung des Rhodiums aus dem Oxorohprodukt erhebliche Schwierigkeiten. Es findet sich nach Beendigung der Umsetzung als Carbonylverbindung im Hydroformylierungsprodukt gelöst.

Zur Aufarbeitung wird das Oxorohprodukt üblicherweise mehrstufig entspannt, indem man den Synthesedruck, d.h. etwa 250 bis 300 bar ($25 \cdot 10^3$ bis $30 \cdot 10^3$ kPa), zunächst auf 15 bis 25 bar ($1{,}5 \cdot 10^3$ bis $2{,}5 \cdot 10^3$ kPa) reduziert. Hierbei wird im Rohprodukt gelöstes Synthesegas frei. Anschließend kann man auf Normaldruck entspannen. Vor der Reindarstellung oder der Weiterverarbeitung des Reaktionsproduktes, die durch Destillation erfolgt, müssen die gelösten Rhodiumverbindungen entfernt werden. Hierbei ist zu berücksichtigen, daß das Edelmetall im Rohprodukt in einer Konzentration von nur wenigen ppm homogen gelöst ist. Zusätzliche Schwierigkeiten können noch dadurch entstehen, daß das Rhodium bei der Entspannung teilweise in metallische Form übergeht oder mehrkernige Carbonyle bildet. In beiden Fällen kommt es dann zur Ausbildung eines heterogenen Systems, das aus der flüssigen organischen Phase und der festen, Rhodium oder Rhodiumverbindungen enthaltenden Phase besteht.

Um die vorstehend beschriebenen Schwierigkeiten zu umgehen extrahiert man nach der Arbeitsweise der US—A—4 292 196 Metalle der VIII. Gruppe des Periodensystems, darunter auch Rhodium aus Hydroformylierungsprodukten mit wäßrigen Lösungen von Stickstoffverbindungen wie Ammoniak oder einem Amin.

Nach dem Verfahren der deutschen Patentanmeldung P 33 47 406.0 wird Rhodium dadurch aus den Produkten der Oxosynthese abgetrennt und wiedergewonnen, daß man es mit Hilfe komplexbildender Reagenzien aus dem Oxorohprodukt extrahiert.

Unter dem Begriff Oxorohprodukt wird dabei das nach Entspannen und gegebenenfalls Abkühlen anfallende Reaktionsgemisch verstanden.

Nach einer bevorzugten Auführungsform der älteren Arbeitsweise setzt man als Komplexbildner Sulfonate und Carboxylate organischer Phosphine der allgemeinen Formel

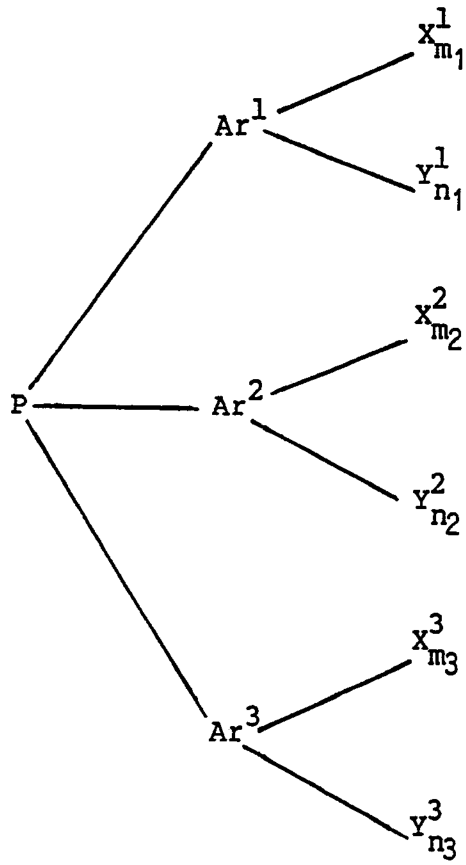

ein. Hierbei bedeuten $Ar^1$, $Ar^2$, $Ar^3$ jeweils eine Phenyl- oder Naphthylgruppe, $Y^1$, $Y^2$, $Y^3$ jeweils eine geradkettige oder verzweigte Alkylgruppe mit 1 bis 4 C-Atomen, eine Alkoxygruppe, ein Halogenatom, die OH—, CN—, $NO_2$— oder $R^1R^2N$-Gruppe, in der $R^1$ und $R^2$ für jeweils eine geradkettige oder verzweigte Alkylgruppe mit 1 bis 4 C-Atomen stehen; $X^1$, $X^2$, $X^3$ ist jeweils ein Carboxylat-($COO^-$—) und/oder ein Sulfonat-($SO^{3-}$—)Rest, $m_1$, $m_2$, $m_3$ sind gleiche oder verschiedenze ganze Zahlen von 0 bis 3, wobei mindestens eine Zahl $m_1$, $m_2$, $m_3$ gleich oder größer als 1 ist; $n_1$, $n_2$, $n_3$ sind gleiche oder verschiedene ganze Zahlen von 0 bis 5. M ist ein Alkalimetallion, ein Äquivalent eines Erdalkalimetall-, oder Zinkions, ein Ammonium- oder quarternäres Ammoniumion der allgemeinen Formel $N(R^3R^4R^5R^6)^+$ in der $R^3$, $R^4$, $R^5$, $R^6$ jeweils für eine geradkettige oder verzweigte Alkylgruppe mit 1 bis 4 C-Atomen steht.

Nach einer bevorzugten Ausführungsform dieses Verfahrens verwendet man als komplexbildende Reagenzien Verbindungen der vorstehend beschriebenen allgemeinen Formel, in der $Ar^1$, $Ar^2$, $Ar^3$ jeweils einen Phenylrest, $X^1$, $X^2$, $X^3$ jeweils einen Sulfonatrest und $m_1$, $m_2$, $m_3$ jeweils die Zahlen 0 oder 1 bedeuten, wobei die Summe von $m_1$, $m_2$ und $m_3$ 1, 2 oder 3 ist.

Die aus Rhodium und den Sulfonaten oder Carboxylaten organischer Phosphine gebildeten Komplexverbindungen sind wasserlöslich. Lementsprechend kann das Rhodium aus dem Oxorohprodukt, also der organischen Phase, mit einer wässrigen Lösung des substituierten Phosphins extrahiert werden.

Das Rhodium geht hierbei in die wässrige Phase über, die sich durch einfaches Dekantieren vom organischen Produktgemisch abtrennen läßt. Durch Kreislaufführung der Lösung des Komplexbildners können hohe Rhodium-Konzentrationen in der wässrigen Phase erreicht werden.

Die vorliegende Erfindung betrifft eine weitere Verbesserung des vorstehend beschriebenen Prozesses.

Sie besteht in einem Verfahren zur Abtrennung und Widergewinnung von als Katalysator ohne Komplexbildner eingesetztem Rhodium durch Extraktion aus einer organischen Lösung der Produkte der Oxosynthese mit einer wäßrigen Lösung organischer Phosphine der allgemeinen Formel

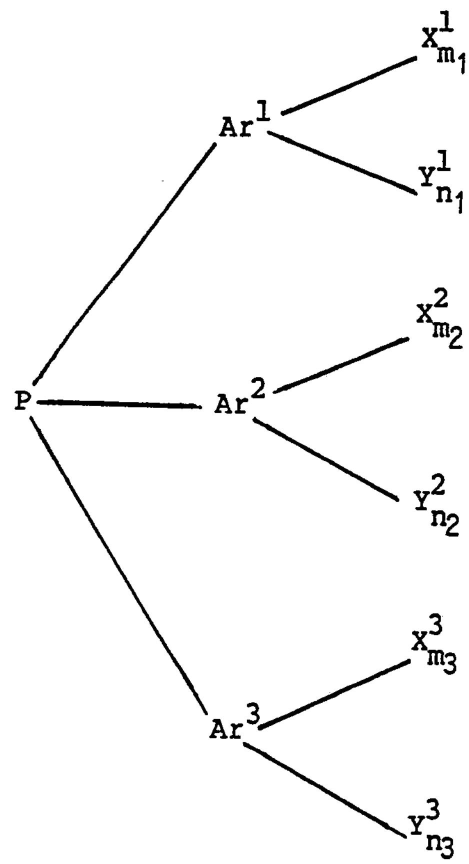

als komplexbildenden Reagenzien, wobei $Ar^1$, $Ar^2$, $Ar^3$ jeweils eine Phenyl- oder Naphthylgruppe, $Y^1$, $Y^2$, $Y^3$ jeweils eine geradkettige oder verzweigte Alkylgruppe mit 1 bis 4 C-Atomen, eine Alkoxygruppe, ein Halogenatom, die OH—, CN—, $NO_2$— oder $R^1R^2N$-Gruppe, in der $R^1$ und $R^2$ für jewils eine geradkettige oder verzweigte Alkylgruppe mit 1 bis 4 C-Atomen stehen, bedeuten, $X^1$, $X^2$, $X^3$ jeweils ein Sulfonat-($SO_3^-$—) und/oder ein Carboxylat-($COO^-$—)Rest ist, $m_1$, $m_2$, $m_3$ gleiche oder verschiedene ganze Zahlen von 0 bis 3 sind und mindestens eine Zahl $m_1$, $m_2$, $m_3$ gleich oder größer als 1 ist, $n_1$, $n_2$, $n_3$ gleiche oder verschiedene ganze Zahlen von 0 bis 5 sind und in den Sulfonaten und Carboxylaten Alkalimetall-, Erdalkalimetall-, Zink-, Ammonium- oder quaternäre Ammoniumionen der allgemeinen Formel $N(R^3R^4R^5R^6)^+$ in der $R^3$, $R^5$, $R^6$ jeweils für eine geradkettige oder verzweigte Alkylgruppe mit 1 bis 4 C-Atomen steht, enthalten sind und ist dadurch gekennzeichnet, daß der wäßrigen Lösung des Komplexbildners ein Lösungsvermittler zugesetz wird.

Überraschenderweise ermöglicht die neue Arbeitsweise eine Beschleunigung und weitere Vervollständigung der Extraktion der Rhodiums aus der organischen und seines Überganges in die wäßrige Phase.

Unter dem Ausdruck Lösungsvermittler werden Stoffe oder Stoffgemische verstanden, die sowohl mit der wäßrigen, als auch mit der organischen Phase verträglich und insbesondere be erhöhten Temperaturen in beiden Phasen löslich sind. Derartige Stoffe sind bekannt und werden auch als Phasentransfer-, oberflächenaktives oder amphiphiles Reagenz oder als Tensid bezeichnet.

Ihre Wirkung besteht insbesondere darin, daß sie die physikalischen Eigenschaften der Grenzflächen zwischen den beiden flüssigen Phasen verändern und dadurch den Überang des wäßrigen Extrationsmittels in die Produktphase und des Rhodiums aus der Produktphase in die wäßrige Komplexbildner-Phase beschleunigen. Durch Mitverwendung eines Lösungsvermittlers wird die Extraktion vereinfacht und der apparative Aufwand verringert. Mit dem neuen Verfahren gelingt es, mehr als 95% des in der Produktphase enthaltenen Rhodiums zurückzugewinnen; es erfüllt damit eine der wichtigsten Voraussetzungen für die technische Durchführung der Hydroformylierung in Gegenwart von Rhodiumkatalysatoren.

Von besonderer Bedeutung ist in diesem Zusammenhang, daß der Lösungsvermittler keinen negativen Einfluß auf die Aktivität des katalytisch wirksamen Metalls hat, so daß besondere Aufarbeitungs- oder Aktivierungsschritte nicht erforderlich sind.

Beispiele für anionische Lösungsvermittler, die in dem erfindungsgemäßen Prozeß eingesetzt werden können, sind Salze von Carbonsäuren mit 8 bis 20 Kohlenstoffatomen, insbesondere von gesättigten Fettsäuren mit 12 bis 18 Kohlenstoffatomen wie Laurin-, Myristin- und Stearinsäure. Zu den anionischen Lösungsvermittlern gehören ferner Alkylsulfonate und Alkylarylsulfonate wie Alkylbenzolsulfonate und Alkylnaphthalinsulfonate.

Als kationische Lösungsvermittler können Amine, derer hockmolekularer rest unmittelbar oder über ein Heteroatom an das Stickstoffatom gebunden ist, verwendet werden. Beispiele für die erste Gruppe der genannten Verbindungen sind Octadecyldiethylamin, Octadecylethanolamin, Lauryldipolyglycolamin und 2-Heptadecylimidazolin-chlorhydrat.

Zur zweiten Gruppe zähalen insbesondere Verbindungen mit hydrolysestabilen Entergruppen wie Octylphenoldiethylaminethylglycolether.

Besonders geeignete kationische Lösungsvermittler sind quarternäre Oniumverbindungen insbesondere Ammoniumsalze. Bewährt haben sich vor allem Verbindungen der allgemeinen Formel

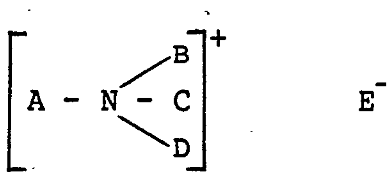

in der A für einen geradkettigen oder verzweigten Alkyl-, Alkoxy-, Hydroxyalkyl- oder einen gegebenenfalls substituierten Arylrest mit jeweils 6 bis 25 Kohlenstoffatomen oder für den Rest $R^7$—CONH—$CH_2$—$CH_2$—$CH_2$, wobei $R^7$ einen geradkettigen oder verzweigten Alkylrest mit 5 bis 11 Kohlenstoffatomen bedeutet, steht. A ist insbesondere ein geradkettiger oder verzweigter Alkylrest mit 8 bis 16 Kohlenstoffatomen oder ein, gegebenenfalls substituierter Arylrest mit 10 bis 14 Kolenstoffatomen. B ist ein geradkettiger oder verzweiger Alkylrest mit 1 bis 25 Kohlenstoffatomen oder ein gegebenenfalls substituierter Arylrest mit 6 bis 25 Kohlenstoffatomen. C und D sind gleich oder verschieden und bedeuten geradkettige oder verzweigte Alkylreste mit 1 bis 4 Kohlenstoffatomen oder bilden zusammen mit N einen heterocyclischen Fünf- oder Sechsring. E steht für Chlorid, Bromid, Jodid und insbesondere Sulfat, Tetrafluoborat, Acetat, Methylsulfat, Benzolsulfonat, Alkylbenzolsulfonat, Toluolsulfonat, Lactat oder Citrat. Bevorzugt sind als Anionen wegen ihres geringen korrosiven Verhaltens die Methylsulfate, Sulfonate und Lactate. Beispiele für geeignete Kationen, die der oben wiedergegebenen Formel folgen, sind Stearyltrimethylammonium, Phenyltrimethylammonium, Benzyltrimethylammonium, Dimethylbenzyldodecylammonium, Cetyltrimethylammonium, Myristyltrimethylammonium, Dodecylpyridinium, Stearoylamidomethylpyridinium, Cetyldimethylbenzylammonium, Distearyldimethylammonium, Lauryltrimethylammonium, Benzyltriethylammonium.

Zu den neutralen oder nichtionogenen Lösungsvermittlern zählen insbesondere die Addukte des Ethylenoxids wie Alkylpolyethylenglykole (erhlaten durch Anlagerung höhermolekularer Alkohole an Ethylenoxid), Alkylphenylpolyethylenglykole (erhalten durch Anlagerung der Phenole an Ethylenoxid) und Acylpolyethylenglykole (erhalten durch Anlagerung von Fettsäuren an Ethylenoxid). Auch polare Lösungsmittel wie Sulfolan und Dimethylsulfoxid sin geeignet.

Die Wahl des geeigneten Lösungsvermittlers hängt von der Zusammensetzung der organischen Phase ab und ist auf sie abzustimmen. Es ist vorteilhaft, wenn er polare und nichtpolare Molekülteile enthält, um die geforderte Aktivität für beide, die wässrige und die organische Phase, zu sichern. Insbesondere soll der Lösungsmittler bevorzugt in der wässrigen Phase und nur zu einem kleineren Teil in der organischen Phase verteilt sein. Die Lösungsvermittler können allein oder als Mischung von zwei oder mehr Substanzen eingesetzt werden.

Die Konzentration des Lösungsvermittlers in der wässrigen Lösung beträgt 0,005 bis 10 Gew.% (bezogen auf die Lösung), vorzugsweise 0,1 bis 2,5 Gew.% Konzentrationen oberhalb 2,5 Gew.% können je nach dem gewählten Lösungsvermittler mehr oder weniger stark die Tendenz zur Schaumbildung begünstigen und dadurch die rasche Phasentrennung beeinträchtigen.

Mit besonderem Erfolg wird das erfindungsgemäße Verfahren zur Abtrennung und Widergewinnung von Rhoidum aus den Produkten der Hydroformylierung end- und innenständiger verzweigter Olefine mit mehr als 5 Kohlenstoffatomen wie i-Hepten, Diisobutylen, Tri- und Tetrapropylen oder dem unter der Bezeichnung Dimersol® im Handel befindlichen Gemisch von $C_8$-Olefinen angewandt. Selbstverständlich kann das Verfahren auch auf die Hydroformylierung unverzweigter end- und mittelständiger Olefine angewendet werden, wobei in der Regel die absoluten Rh-Konzentrationen niedriger liegen.

Die nach der Phasentrennung zurückbleibende, vom Rhodium befreite organische Produktphase wird zur Entfernung restlichen Extraktionsmittels, Rhodiums und Lösungsvermittlers mit Wasser gewaschen und denach der üblichen destillativen Produktaufarbeitung zugeführt. Das Waschwasser kann im Kreis geführt werden. Ein Teilstrom kann zur Ergänzung der Wasserluste in die Extraktionsstufe geleitet werden um ein Aufkonzentrieren der Komplexbildner-Lösung zu verhindern, da das Oxorohprodukt laufend eine geringe Wasermenge aus der Komplexbildner-Lösung entnimmt. Diese Wassermenge wird in der Waschstufe als Frischwasserzusatz ergänzt.

Die wässrige, Rhodium in hoher Konzentration enthaltende Phase wird dem Reaktionsgemisch entweder unmittelbar oder gereinigt und aufkonzentriet als Katalysatorlösung zugeführt. Es ist auch möglich, das Rhodium als in Wasser schwer- oder unlösliche Verbindung, z.B. in Form des Rhodium-2-ethylhexanoats abzuscheiden und erneut als Katalysator einzusetzen.

In den nachstehenden Beispielen werden verschiedene Ausführungsformen der Erfindung beschrieben, das beanspruchte Verfahren ist jedoch nicht auf diese Ausführungsformen beschränkt.

Beispiele

In den Beispielen 1 bis 8 wird jeweils auf 20 bis 25°C abgekühlter und mehrere Stunden gelagerter roher Isooctylaldehyd, erhalten durch Hydroformylierung von i-Hepten, eingesetzt. Die Beispiele 1, 3, 5, 7 betreffen die Rhodium-Extraktion ohne Zusatz eines Lösungsvermittlers; in den Beispielen 2, 4, 6, 8 wird ein Lösungsvermittler mitverwendet. In den Beispielen 9 bis 12 wird die Abtrennung von Rhodium aus verschiedenen Hydroformylierungsprodukten beschrieben, wobei in den Beispielen 9 und 11 ohne Lösungsvermittler gearbeitet wird.

Die Abkürzung TPPTS steht für Triphenylposphintrisulfonat. Alle Konzentrationsangaben erfolgen in Gew.%

Beispiel 1 (Vergleich)

In einem Rührkolben werden 200 g roher Isooctylaldehyd, der
34,9% $C_7$-Kohlenwasserstoff (überwiegend Hepten)
62,7% Isooctylaldehyd
2,2% Isooctylakohol
0,2% Höhersieder
und 3,9 ppm Rhodium enthält mit 20 g einer O,1 prozentigen wässrigen Natrium-TPPTS-Lösung versetzt. Das molare Verhältnis von Phosphor zu Rhodium beträgt 5. Beide Phasen werden 5 Minuten bei 50°C intensiv gerührt. Nach Beendigung des Rührens trennen sich die beiden Phasen innerhalb von 12 Sekunden ohne Emulsionsbildung. Die organische Oxorohpruduktphase enthält noch 1,1 ppm Rhodium, entsprechend einer Rhodiumabtrennung von 72%.

Beispiel 2

Es wird wie in Beispiel 1 verfahren mit dem Unterschied, daß der wässrigen TPPTS-Lösung 0.1 g Cetyltrimethylammoniummethosulfat zugesetzt und eine Minute gerührt wird. Die organische Oxorohprodukt-Phase enthält lediglich noch 0.6 ppm Rhodium, entsprechend einer Rhodiumabtrennung von 85%.

Beispiel 3 (Vergleich)

Es wird wie in Beispiel 1 gearbeitet, jedoch unter Verwendung einer 0.4 prozentigen Natrium-TPPTS-Lösung. Das molare Verhältnis von Phosphor zu Rhodium beträgt 20. In der organischen Phase bleibt 1 ppm Rhodium zurück, entsprechend einer Rhodiumabtrennung von 74%.

Beispiel 4

Es wird wie in Beispiel 3 gearbeitet mit dem Unterschied, daß der wässrigen TPPTS-Lösung 0.1 g Dodecyltrimethylammoniumsulfat zugesetzt und eine Minute gerührt wird. In der organischen Phase verbleiben 0.6 ppm Rhodium, entsprechend einer Rhodiumabtrennung von 85%.

Beispiel 5 (Vergleich)

Es wird wie in Beispiel 3 gearbeitet, jedoch bei einer Temperatur von 80° (statt 50°C) gerührt. Die Trennung der beiden Phasen erfolgt in 9 Sekunden. Im Rohprodukt bleibt 1 ppm Rhodium zurück, entsprechend einer Rhodiumabtrennung von 74%.

Beispiel 6

Es wird wie in Beispiel 5 gearbeitet, jedoch unter zusätzlicher Verwendung von 0.1 g Pyridiniumsulfat und einer Rührzeit von einer Minute. Die Phasentrennung erfolgt in 9 Sekunden. Im Rohprodukt bleiben 0.5 ppm Rhodium zurück, entsprechend einer Rhodiumabtrennung von 87%.

Beispiel 7 (Vergleich)

In einem Rundkolben mit Bodenablaß, Gaseinleitungskapillare und Rührer werden 1 000 g Isooctylaldehyd der in Beispiel 1 genannten Zusammensetzung mit jeweils 100 g 20 prozentiger Natrium-TPPTS-Lösung extrahiert. Über die Gaseinleitungskapillare wird zunächst Synthesegas ($CO/H_2 = 1:1$) zur Sättigung der Mischung mit CO und Wasserstoff eingeleitet und anschließend bei 80°C intensiv gerührt. Die Rührzeit sowie die anschließende Ruhezeit betragen 30 Sekunden. Darauf wird die wässrige Phase über den Bodenablaß ausgeschleust und die organische Phase erneut mit 100 g einer 20 prozentigen Natrium-TPPTS-Lösung behandelt. Insgesamt wird viermal extrahiert. Die organische Phase enthält danach nur noch 0.6 ppm Rhodium, entsprechend einer Rhodiumabtrennung von 85%.

Beispiel 8

Es wird entsprechend Beispiel 7 gearbeitet mit dem Unterschied, daß die Extraktionsschritte mit einer TPPTS-Lösung erfolgen, die als Zusatz 2% Benzyltrimethylammoniumsulfat enthalten und die Rührzeit nur 20 Sekunden beträgt. Nach Abschluß der Extraktionen weist die organische Phase noch 0.3 ppm Rhodium auf, entsprechend einer Rhodiumabtrennung von 92%.

Beispiel 9 (Vergliech)

In der Apparatur des Beispiels 7 werden 1 000 g roher Propionaldehyd, der

96,3% Propionaldehyd
0,2% n-Propanol
1,4% Ethylen + Ethan
2,1% Höhersieder

und 9,6 ppm Rhodium enthält jeweils 100 g 20 prozentiger Natrium-TPPTS-Lösung in 5 Extraktionsschritten bei 86°C behandelt. Der Rhodiumgehalt der organischen Phase beträgt nach der 1. Extraktion 1 ppm, entsprechend einer Rhodiumabtrennung von 66% und nach der 5. Extraktion 0,6 ppm, entsprechend einer Rhodiumabtrennung von 91%.

Beispiel 10

Es wird wie in Beispiel 9 gearbeitet mit dem Unterschied, daß der wässrigen TPPTS-Lösung 2% Cetyltrimethylammoniumacetat zugesetzt wird. Der Rhodiumgehalt der organischen Phase nach der 1. Extraktion beträgt 0.6 ppm, entsprechend einer Rhodiumabtrennung von 91% nach der 5. Extraktion 0.2 ppm, entsprechend einer Rhodiumabtrennung von 97%.

Entsprechend verbesserte Ergebnisse erhält man auch bei kontinuierlicher Durchführung.

Beispiel 11 (Vergleich)

450 g eines Rückstandes aus der Hydroformylierung eines $C_{20}$—$C_{40}$-α-Olefingemisches mit einem Rhodiumgehalt von 17 ppm werden im Autoklaven bei 100°C mit 50 g einer 20 prozentigen TPPTS-Lösung extrahiert. Nach Abkühlung und Phasentrennung beträgt der Rhodiumgehalt der nicht destillierbaren organischen Phase 10 ppm, entsprechend einer Rhodiumextraktion von 41%.

Beispiel 12

Versuch 11 wird mit einer wässrigen TPPTS-Lösung weiderholt, die 2,5% Tetradecyltrimethylammoniumlactat (bezogen auf die Lösung) enthält. Der Rhodiumgehalt der organischen Phase beträgt nach der Extraktion 0.2 ppm, entsprechend einer Rhodium-Rückgewinnung von 99%.

Die Beispiele zeigen, daß durch Zusatz eines Lösungsvermittlers die Rhodium-Extraktion schneller und durchgreifender verläuft. Aus Beispiel 12 geht hervor, daß auch nicht destillierbare Oxorohprodukte auf schonende Weise vom Rhodium-Katalysator befreit werden können.

**Patentansprüche**

1. Verfahren zur Abtrennung und Wiedergewinnung von als Katalysator ohne Komplexbildner eingesetztem Rhodium durch Extraktion aus einer organischen Lösung der Proudkte der Oxosynthese mit einer wäßrigen Lösung organischer Phopshine der allgemeinen Formel

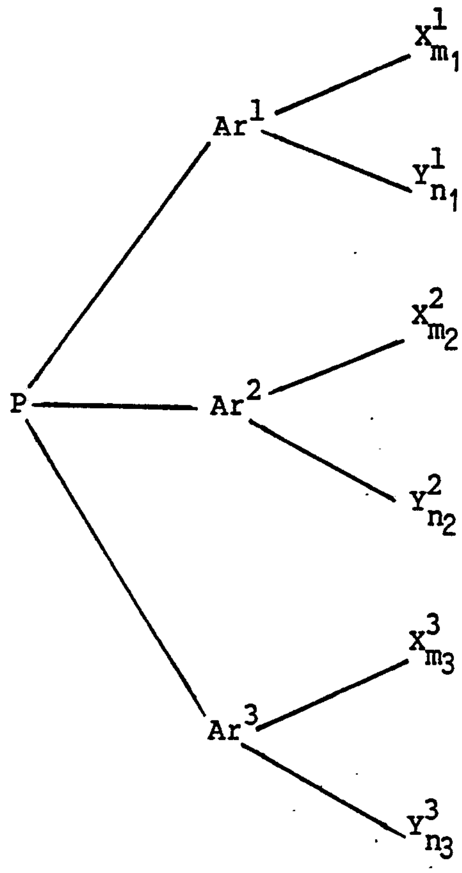

als komplexbildenden Reagenzien, wobei $Ar^1$, $Ar^2$, $Ar^3$ jeweils eine Phenyl- oder Naphthylgruppe, $Y^1$, $Y^2$, $Y^3$ jeweils eine geradkettige oder verzweigte Alkylgruppe mit 1 bis 4 C-Atomen, eine Alkoxygruppe, ein Halogenatom, die OH—, CN—, $NO_2$— oder $R^1R^2N$-Gruppe, in der $R^1$ und $R^2$ für jeweils eine geradkettige oder verzweigte Alkylgruppe mit 1 bis 4 C-Atomen stehen, bedeuten, $X^1$, $X^2$, $X^3$ jeweils ein Sulfonat-($SO^{3-}$—) und/oder ein Carboxylat-($COO^-$—) Rest ist, $m_1$, $m_2$, $m_3$ gleiche oder vershiedene ganze Zahlen von 0 bis 3 sind und mindestens eine Zahl $m_1$, $m_2$, $m_3$ gleich oder größer als 1 ist, $n_1$, $n_2$, $n_3$ gleiche oder verschiedene ganze Zahlen von 0 bis 5 sind und in den Sulfonaten und Carboxylaten Alkalimetall-, Erdalkalimetall-, Zink-, Ammonium- oder quaternäre Ammoniumionen der allgemeinen Formel $N(R^3R^4R^5R^6)^+$ in der $R^3$, $R^4$, $R^5$, $R^6$ jeweils für eine geradkettige oder verzweigte Alkylgruppe mit 1 bis 4 C-Atomen steht, enthalten sind, dadurch gekennzeichnet, daß der wäßrigen Lösung des Komplexbildners ein Lösungsvermittler zugesetzt wird.

2. Verfahren nach Anspruch 1, dadurch gekennzeichnet, daß als Lösungsvermittler Verbindungen der allgemeinen Formel

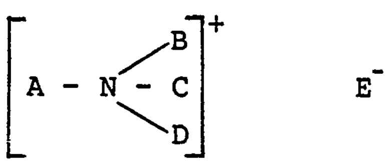

eingesetzt werden, in der A für Wasserstoff, einen geradkettigen oder verzweigten Alkyl-, Alkoxy-, Hydroxyalkyl- oder einen gegebenenfalls substituierten Arylrest mit 6 bis 25 Kohlenstoffatomen oder für den Rest $R^7$—CONH—$CH_2$—$CH_2$—$CH_2$, wobei $R^7$ ein geradkettiger oder verzweigter Alkylrest mit 5 bis 11 Kohlenstoffatomen ist, steht, B ein geradkettiger oder verzweigter Alkylrest mit 1 bis 25 Kohlenstoffatomen oder ein gegebenenfalls substituierter Arylrest mit 6 bis 25 Kohlenstoffatomen ist, C und D gleich oder verschieden sind und geradkettige oder verzweigte Alkylreste mit 1 bis 4 Kohlenstoffatomen bedeuten, zusammen mit N einen heterocyclisschen Fünf- oder Sechsring bilden oder B, C und D und das Stickstoffatome der Pyrindinring sind und E für Chlorid, Bromid, Jodid und insbesondere Sulfat, Tetrafluoborat, Acetat, Methylsulfat, Benzolsulfonat, Alkylbenzolsulfonat, Toluolsulfonat, Lactat oder Citrat steht.

3. Verfahren nach Anspruch 2, dadurch gekennzeichnet, daß A für einen geradkettigen oder verzweigten Alkylrest mit 8 bis 16 Kohlenstoffatomen oder einen, gegebenenfalls substituierten Arylrest mit 10 bis 14 Kohlenstoffatomen steht.

4. Verfahren nach Anspruch 2, dadurch gekennzeichnet, daß C und D zusammen mit dem Stickstoffatom für Pyrrol oder Morpholin stehen.

5. Verfahren nach Anspruch 1 bis 4, dadurch gekennzeichnet, daß die Konzentration des Lösungsvermittlers in der wässrigen Lösung 0,005 bis 10 Gew.-%, vorzugsweise 0,1 bis 2,5 Gew.-% (jeweils bezogen auf die Lösung), beträgt.

**Revendications**

1. Procédé pour la séparation et la récupération du rhodium utilisé comme catalyseur sans agent complexant par extraction d'une solution organique des produits de l'oxosynthèse par une solution aqueuse de phosphines organiques de formule générale:

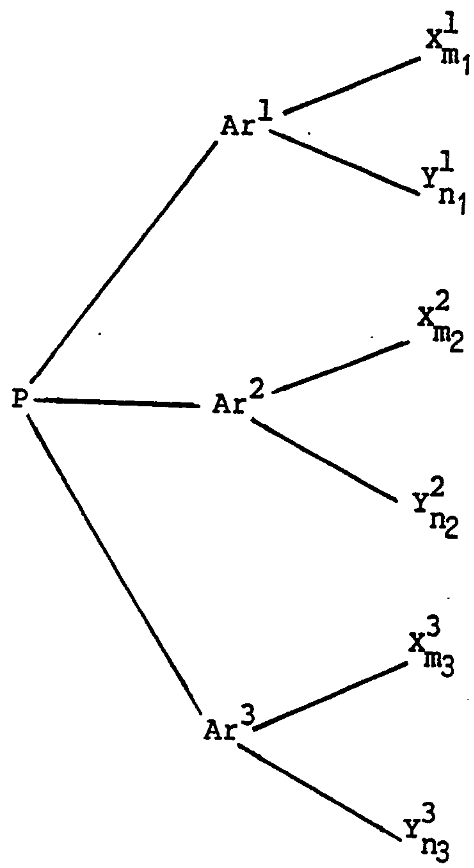

comme réactifs complexants, dans laquelle $Ar^1$, $Ar^2$ et $Ar^3$ représentent chacun un groupe phényle ou naphtyle, $Y^1$, $Y^2$, et $Y^3$ représentent chacun un groupe alkyle à chaîne droite ou ramifiée en $C_1$—$C_4$, un groupe alcoxy, un atome d'halogène, le groupe OH, le groupe CN, le groupe $NO_2$ ou le groupe $R^1R^2N$ dans lequel $R^1$ et $R^2$ représentent chacun un groupe alkyle à chaîne droite ou ramifiée en $C_1$—$C_4$, $X^1$, $X^2$ et $X^3$ représentent chacun un reste sulfonate (—$SO_3^-$) et/ou un reste carboxylate (—$COO^-$), $m_1$, $m_2$ et $m_3$ sont des nombres entiers égaux ou différents de 0 à 3 et l'un au moins des nombres $m_1$, $m_2$ et $m_3$ est égal ou supérieur à 1, $n_1$, $n_2$ et $n_3$ sont des nombres entiers égaux ou différents de 0 à 5 et les sulfonates et carboxylates contiennent des ions de métaux alcalins ou alcalino-terreux, de zinc ou d'ammonium ou de ions ammonium quaternaires de formule générale $N(R^3R^4R^5R^6)^+$ dans laquelle $R^3$, $R^4$, $R^5$ et $R^6$ représentent chacun un groupe alkyle à chaîne droite ou ramifiée en $C_1$—$C_4$, caratérisé en ce que l'on ajoute un agent de solubilisation à la solution aqueuse de l'agent complexant.

2. Procédé selon la revendication 1, caractérisé en ce que l'on utilise comme agents de solubilisation des composés de formule générale:

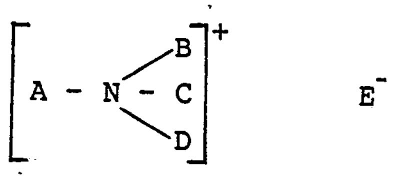

dans laquelle A représente l'hydrogène ou un groupe alkyle, alcoxy ou hydroxyalkle à chaîne droite ou ramifiée ou un reste aryle éventullement substitué en $C_6$—$C_{25}$ ou le reste $R^7$—CONH—$CH_2$—$CH_2$—$CH_2$ dans lequel $R^7$ représente un reste alkyle à chaîne droite ou ramifiée en $C_5$—$C_{11}$, B est un reste alkyle à chaîne droite ou ramifiée en $C_1$—$C_{25}$ ou un reste aryle en $C_6$—$C_{25}$ éventuellement substitué, C et D sont identiques ou différents et représentent des restes alkyles à chaîne droite ou ramifiée en $C_1$—$C_4$, ou forment avec l'atome d'azote un noyau hétérocyclique à 5 ou 6 chaînons ou bien B, C et D forment avec l'atome d'azote le noyau pyridine et E représente un anion chlorure, bromure, iodure et en particulier sulfate, tétrafluoborate, acétate, méthylsulfate, benzènesulfonate, alkylbenzènesulfonate, toluènesulfonate, lactate ou citrate.

3. Procédé selon la revendication 2, caractérisé en ce que A représente un reste alkle à chaîne droite ou ramifiée en $C_8$—$C_{16}$ ou un reste aryle en $C_{10}$—$C_{14}$ éventuellement substitué.

4. Procédé selon la revendication 2, caractérisé en ce que C et D forment ensemble avec l'atome d'azote un noyau pyrrole ou morpholine.

5. Procédé selon les revendications 1 à 4, caractérisé en ce que la concentration de l'agent de solubilisation dans la solution aqueuse et de 0,005 à 10% en poids, de préférence de 0,1 à 2,5% en poids (rapportée chaque fois à la solution).

**Claims**

1. A process for separating and recovering rhodium used as a catalyst without a complexing agent by extraction from an organic solution of the products of the Oxo synthesis with an aqueous solution of organic phosphines having the general formula

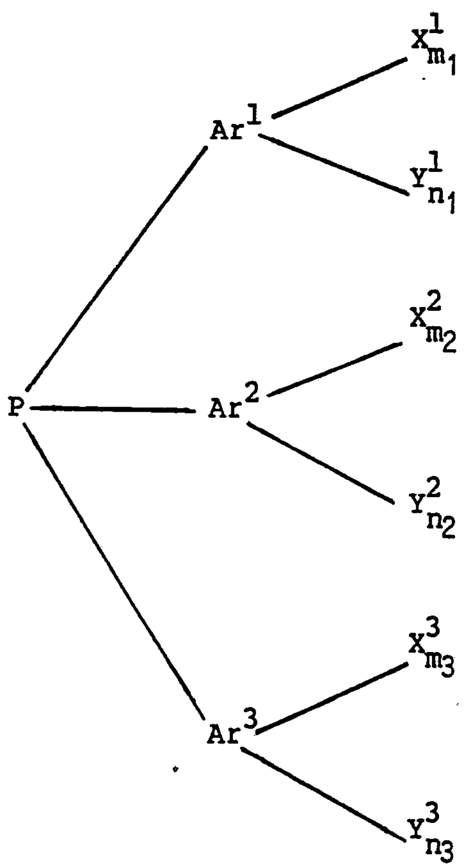

as complexing reagents, where $Ar^1$, $Ar^2$ and $Ar^3$ each denote a phenyl or naphthyl group, $Y^1$, $Y^2$ and $Y^3$ each stand for a straight-chain or branched alkyl group having 1 to 4 carbon atoms, an alkoxy group, a halogen atom, the OH, CN, $NO_2$— or $R^1R^2N$ group, $R^1$ and $R^2$ each standing for a straight-chain or branched alkyl group having 1 to 4 carbon atoms, $X^1$, $X^2$ and $X^3$ are each a sulfonate ($SO_3^-$) and/or a carboxylate ($COO^-$) radical, $m_1$, $m_2$ and $m_3$ are the same or different integers from 1 to 3 and at least one number $m_1$, $m_2$ or $m_3$ is equal to or greater than 1, $n_1$, $n_2$ and $n_3$ are the same or different integers from 0 to 5 and alkali metal, alkaline earth metal, zinc, ammonium or quaternary ammonium ions of the general formula $N(R^3R^4R^5R^6)^+$, $R^3$, $R^4$, $R^5$, $R^6$ each denoting a straight-chain or branched alkyl group having 1 to 4 carbon atoms are contained in the sulfonates and carboxylates, characterised in that a solubiliser is added to the aqueous solution of the complexing agent.

2. A process according to claim 1, characterised in that compounds of the general formula

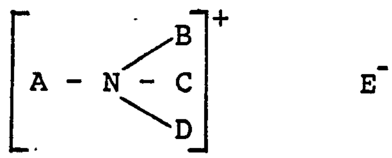

are used as solubilisers, where A stands for hydrogen, a straight-chain or branched alkyl, alkoxy, hydroxyalkyl or an optionally subsituted aryl radical having 4 to 25 carbon atoms or for the radical $R^7$—CONH—$CH_2$—$CH_2$—$CH_2$, $R^7$ being a straight-chain or branched alkyl radical with 5 to 11 carbon atoms, B being a straight-chain or branched alkyl radical having 1 to 25 carbon atoms or an optionally substituted aryl radical having 6 to 25 carbon atoms, C and D are the same or different and represent straight-chain or branched alkly radicals having 1 to 4 carbon atoms, together with N form a heterocyclic five or six-membered ring or B, C and D and the nitrogen atom are the pyridine rings, and E stands for chloride, bromide, iodide and in particular sulfate, tetrafluoroborate, acetate, dimethyl sulfate, benzenesulfonate, alkylbenzene sulfonate, toluenesulfonate, lactate or citrate.

3. A process according to claim 2, characterised in that A stands for a straight-chain or branched alkyl radical having 8 to 16 carbon atoms or an optionally substituted aryl radical having 10 to 14 carbon atoms.

4. A process according to claim 2, characterized in that C and D together with the nitrogen atom stand for pyrrole or morpholine.

5. A process according to claims 1 to 4, characterised in that the concentration of the solubiliser in the aqueous solution is 0.005 to 10% by weight, preferably 0.1 to 2.5% by weight (in each case related to the solution).